# EUROPEAN PATENT APPLICATION

(11) **EP 1 473 566 A1**
(43) Date of publication of application: **03.11.2004**
(21) Application number: 02788770.2
(22) Date of filing: 10.12.2002
(51) Int. Cl.: G01N 33/58, G01N 33/68, G01N 33/50, B82B 1/00

(54) **METHODS FOR PRODUCING LABELED NUCLEIC ACIDS OR PROTEINS**

(30) Priority: 29.10.2002 JP 2002314658
(71) Applicant: NATIONAL INSTITUTE OF AGROBIOLOGICAL SCIENCES, Tsukuba-shi, Ibaraki-ken 305-8602 (JP)
(72) Inventor: OONO, Kiyoharu, Kobe-shi, Hyogo 657-8501 (JP); KOMATSU, Setsuko, Tsukuba-shi, Ibaraki 305-8602 (JP); TAKAIWA, Fumio, Tsukuba-shi, Ibaraki 305-8602 (JP)
(74) Representative: Teipel, Stephan, Dr.
(86) International application number: PCT/JP2002/012917
(87) International publication number: WO 2004/040315

(57) **Abstract**

It was found that nucleic acids or proteins can be effectively differentiated and identified, without being particularly restricted by the number of types of those nucleic acids or proteins, by binding them to a large scale integrated circuit (LSI) which is externally writable and readable without contact, and recording the nucleic acid or protein information on the LSI.

## Description

### Technical Field

The present invention relates to methods for producing labeled nucleic acids or proteins, and more specifically methods which do not depend on isotopes or dyes.

### Background Art

Conventionally, radioisotopes such as ³²P, ¹⁴C, ³H, ¹²⁵I were used to label nucleic acids and proteins as a means for differentiating and identifying those nucleic acids and proteins. Also, beads that generate a characteristic fluorescence, for example Luminex100, have been used to label nucleic acids as a means of differentiating and identifying a large variety of nucleic acids.

However, in methods that use florescence, the maximum number of differentiations is dependent on the types of fluorescent beads that generate the different types of fluorescence. Thus, to differentiate between 100 or more varieties of nucleic acids, it was necessary to develop 100 or more varieties of beads, each variety generating a different type of fluorescence. Consequently, it was impossible to use these methods to label, each differently, the 50, 000 varieties of DNA clones or the 10,000 varieties of proteins in rice for example, and the number of differentiations was limited.

### Disclosure of the Invention

The present invention was made to solve such problems. An objective of this invention is to effectively differentiate nucleic acids or proteins without being restricted by the number of types.

As a result of their dedicated research into solving the above problems, the present inventors found that it is possible to effectively differentiate and identify a wide variety of nucleic acids and proteins of 10,000 kinds or more, by binding the nucleic acids or proteins to a large scale integrated circuit (LSI) that is externally writable and readable without contact, and recording the nucleic acid or protein information on the LSI. The methods of the present invention do not use substances for labeling, as conventional methods do, but are characterized by using information for labeling. Thus there is no particular limit as to the type of substances that can be labeled, and easy labeling is possible by recording on the LSI. Accordingly, even large quantities of nucleic acids or proteins found as a result of genomic analysis can be fully and effectively differentiated using the present invention. The present invention specifically provides:
(1) a method for producing a labeled nucleic acid or protein, wherein the method comprises binding the nucleic acid or protein to an LSI, and recording specific information on the LSI;
(2) the method of (1), wherein the specific information is characteristic to the nucleic acid or protein bound to the LSI;
(3) the method of (1) , wherein a substrate mediates the binding of the nucleic acid or protein to the LSI;
(4) the method of (3), wherein the substrate is selected from the group consisting of cellulose vinyl acetate, α-cyanoacrylate, silicon denatured polymer, epoxy resin, and calcium sulfate;
(5) the method of (1), wherein an antibody bound to the protein mediates the binding of the protein to the LSI; and
(6) the method of (1), wherein a sugar chain of the protein is attached to the LSI and a characteristic of the sugar chain of the protein is described on the LSI.

The present invention provides new methods for producing labeled nucleic acids or proteins. The methods of the present invention are characterized by binding nucleic acids or proteins to an LSI, and recording specific information on the LSI.

In the present invention, the term "LSI" means an LSI chip that consists of an IC that performs transmission control, a microcomputer nonvolatile memory, and so on. As long as the "LSI" used in the present invention is externally writable and readable, it is not particularly limited. In order to differentiate from tens of thousands to millions of molecules, the LSI memory preferably comprises more than 320 million bits. For easy use, the LSI used in the present invention is preferably minute. An example of an LSI on the market is the IC card or the µ-chip (Hitachi). A non-contact type IC card can also be used in the present invention.

In the present invention, the term "nucleic acid" means a _{DNA} or RNA polynucleotide, and so on. The nucleic acids used in the present invention can be single-stranded or double-stranded, linear or circular. These nucleic acids can be of natural origin or artificially-synthesized. Also, the DNAs can be cDNAs or genomic DNAs. The nucleic acid molecules used in the present invention may be inserted in a vector.

Also, in the present invention, the term "protein" means amino acids that are linked to each other by peptide bonds. Accordingly, the proteins of the present invention also include so-called oligopeptides and polypeptides, as well as complete proteins. Furthermore, the peptides can be naturally-occurring or artificially-synthesized.

There are no particular limits to the form of binding between LSIs and nucleic acids or proteins, as long as binding stability can be maintained, e.g., covalent bonding, van der Waal's forces, hydrogen bonding, and so on. An LSI and a protein can be bound for example, by joining an amino group, carboxyl group, or avidin group of the protein to the LSI by the solid phase method.

In the present invention the "binding" of LSIs and nucleic acids or proteins can be direct or indirect. Accordingly, for example, a substrate can also mediate the binding of LSIs and nucleic acids or proteins. Substrates are preferably hydrophilic, and for example, cellulose vinyl acetate, α-cyanoacrylate, a silicon denatured polymer, epoxy resin, and calcium sulfate can be used in the present invention. Even hydrophobic substrates are suitable for use in the present invention as long as they can be changed to hydrophilic substrates in subsequent processes. For example, fluorocarbon resins are hydrophobic, but produce a carboxylic acid when laser treated, becoming hydrophilic. Thus, they can be applied in the present invention. A substrate and an LSI, or a substrate and a nucleic acid or protein can be bound, for example, using absorption or embedding techniques.

When labeling proteins, compounds that comprise a specific affinity to the proteins, for example, antibodies, can be used as well as substrates. As long as the antibodies are specific to the proteins, their forms are not particularly limited and polyclonal antibodies can be used, as well as monoclonal antibodies. Single-chain antibodies or antibody fragments can also be used.

In the present invention, nucleic acids or proteins are labeled by recording "specific information" on the LSI to which they are bound. There is no particular limit as to the specific information, as long as it can be differentiated. For example, the information can also be characteristic of individual nucleic acids and proteins. Characteristics of nucleic acids or proteins include chain length, number of chains, molecular weight, sugar chain properties, activities, functions, and origin, but they are not limited thereto.

Protein or nucleic acid differentiations can be easily performed by the present invention because the information recorded on an LSI is readable without direct contact, using electromagnetic waves. Also, an LSI can be used to read groups of nucleic acids or proteins as a cluster of electromagnetic waves. Consequently, groups of nucleic acids or proteins can be simply classified without being arrayed on sheets of glass and so on, as in conventional DNA and protein arrays.

### Brief Description of the Drawings

Fig. 1 is a photo of an electrophoresis showing the phosphorylase recovery rate when an LSI and phosphorylase are bound using various substrates.
Fig. 2 is a photo of an electrophoresis showing the albumin recovery rate when an LSI and albumin are bound using various substrates.

### Best Mode for Carrying Out the Invention

Herein below, the present invention will be specifically described using examples, however, it is not to be construed as being limited thereto.

### [Example 1] Preparation of LSI-Labeled Nucleic Acids

Substrates, cellulose vinyl acetate (1), α-cyanoacrylate (2), a silicon denatured polymer (3), epoxy resin (4), and calcium sulfate (5) were attached to an LSI. Glutelin cDNA or ribosomal genes were then added, and the LSI was refrigerated for three months. The nucleic acids were then recovered using 100 ml of a lysis buffer solution (1 mM EDTA, 0.5 M NH₄OAc, 10 mM MgCl₂, and 0.1% SDS), and concentrated by 70% ethanol precipitation. Recovery rates were confirmed by 1% agarose gel electrophoresis. In the case of calcium sulfate (5), nucleic acids were not recovered because they were absorbed. However, recovery rates of about 30% were possible for the other substrates.

### [Example 2] Preparation of LSI-Labeled Proteins

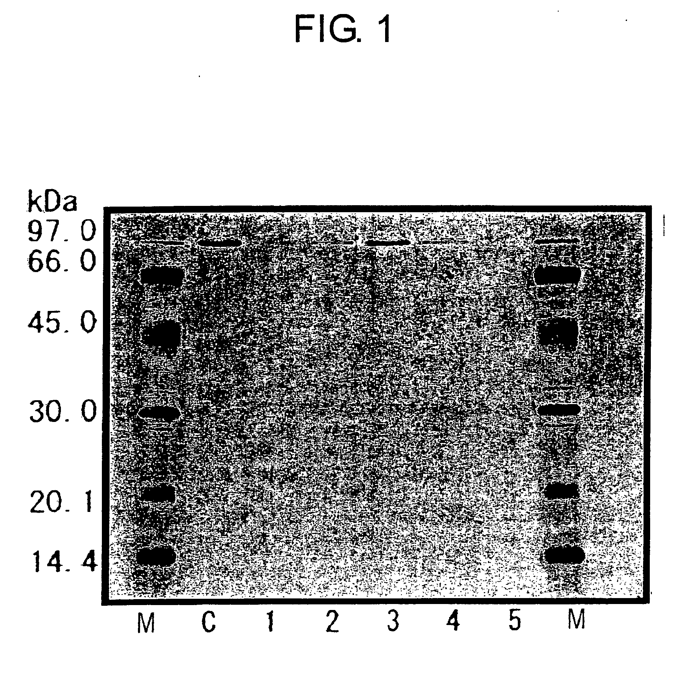

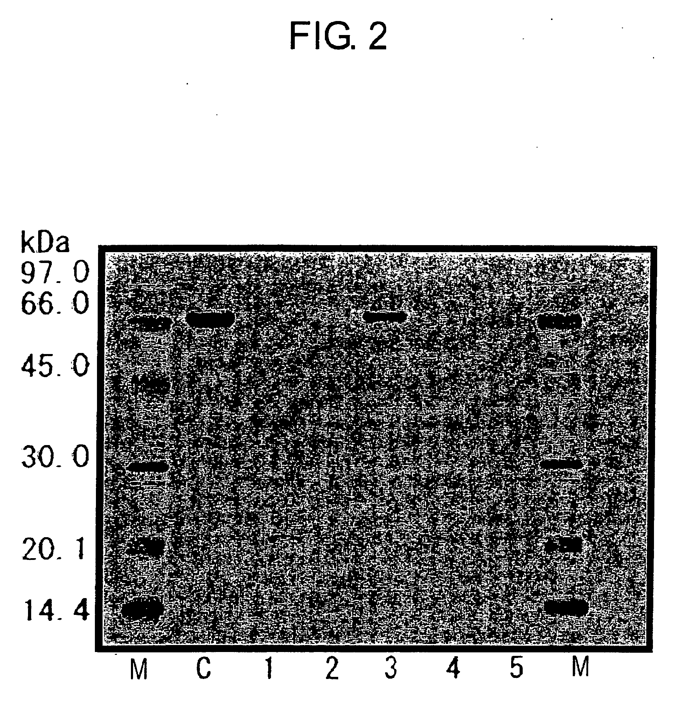

Substrates, cellulose vinyl acetate (1), α-cyanoacrylate (2), a silicon denatured polymer (3), epoxy resin (4), and calcium sulfate were attached to an LSI. Purified protein phosphorylase or 5 ml (1 mg) of bovine serum albumin was added, and the LSI was refrigerated for three months. The proteins were then recovered using 5 ml of a sample buffer solution (SDS-sample buffer), and subjected to polyacrylamide gel electrophoresis (SDS-PAGE). Recovery rates were confirmed by Coomassie brilliant blue staining and destaining (Figs. 1 and 2). In the figures, C shows the target, and 1 mg of the purified protein phosphorylase or albumin was directly separated by SDS-PAGE. In the case of the silicon denatured polymer (3), the phosphorylase recovery rate was 100%, and that of albumin was 90%.

### Industrial Applicability

Conventionally, the differentiation of a number of kinds of nucleic acids or proteins required the development of different signal-emitting "substances", according to the number of those kinds. However, in the present invention, different "information" of the nucleic acids or proteins to be differentiated is recorded on the same substance, called an LSI. Differentiation is performed based on differences in the information. Therefore, in theory, as long as there are unlimited varieties of information, the number of nucleic acids or proteins that can be differentiated is also unlimited. Furthermore, since differentiation is performed using information content, it is simple and certain, and there is also the advantage that constant recognition is possible. Accordingly, the present invention makes it possible to dramatically improve the effectiveness of labeling, as well as the varieties of nucleic acids and proteins which can be labeled.

## Claims

1. A method for producing a labeled nucleic acid or protein, wherein the method comprises binding the nucleic acid or protein to an LSI, and recording specific information on the LSI.

2. The method of claim 1, wherein the specific information is characteristic to the nucleic acid or protein bound to the LSI.

3. The method of claim 1, wherein a substrate mediates the binding of the nucleic acid or protein to the LSI.

4. The method of claim 3, wherein the substrate is selected from the group consisting of cellulose vinyl acetate, α-cyanoacrylate, silicon denatured polymer, epoxy resin, and calcium sulfate.

5. The method of claim 1, wherein an antibody bound to the protein mediates the binding of the protein to the LSI.

6. The method of claim 1, wherein a sugar chain of the protein is attached to the LSI, and a characteristic of the sugar chain of the protein is described on the LSI.
